# EUROPEAN PATENT SPECIFICATION

(11) **EP 2 968 974 B1**
(45) Date of publication and mention of the grant of the patent: **03.05.2017**
(21) Application number: 14710548.0
(22) Date of filing: 14.03.2014
(51) Int. Cl.: A61N 5/10

(54) **INTRA-FRACTION MOTION MANAGEMENT SYSTEM**
SYSTEM FÜR BRUCHINTERNE BEWEGUNGSVERWALTUNG
SYSTÈME DE GESTION DES MOUVEMENTS INTRA-FRACTION

(30) Priority: 15.03.2013 US 201313835685
(43) Date of publication of application: 20.01.2016
(73) Proprietor: Elekta AB (PUBL)., 103 93 Stockholm (SE)
(72) Inventor: LIDSTRÖM, Mattias, 192 55 Sollentuna (SE); FRENCH, Malcolm, 116 38 Stockholm (SE); GORKA, Bartosz, 170 69 Solna (SE); CHEN, Rui, 112 53 Stockholm (SE); ARN, Thomas, 181 46 Lidingö (SE)
(74) Representative: Groth & Co. KB
(86) International application number: PCT/EP2014/055085
(87) International publication number: WO 2014/140262

(56) References cited:
- WO-A1-97/40766
- WO-A1-98/17177
- WO-A2-2004/049109
- US-A- 5 622 187
- US-B1- 7 024 237

## Description

### Field of the invention

The present invention relates to the field of radiation therapy. In particular, the invention concerns systems for monitoring intra-fraction motions of patients in connection with radiation therapy systems.

### Background of the invention

The development of surgical techniques has made great progress over the years. For instance, for patients suffering from cancer tumors requiring surgery, non-invasive surgery is now available which is afflicted with very little trauma to the patient.

One system for external beam radiotherapy is sold under the name of Leksell Gamma Knife^{®}, which provides such surgery by means of gamma radiation. The radiation is emitted from a large number of fixed radioactive sources and is focused by means of collimators, i.e. passages or channels for obtaining a beam of limited cross section, towards a defined target or treatment volume. During treatment, each of the sources provides a dose of gamma radiation insufficient to damage intervening tissue. However, tissue destruction occurs where the radiation beams from all radiation sources intersect or converge, causing the radiation to reach tissue-destructive levels. The point of convergence is hereinafter referred to as the "focus point". Such a radiation device is, for example, referred to and described in US 4,780,898.

Another system for non-invasive surgery is a linear accelerator (LINAC), which also can be used in stereotactic radiosurgery similar to that achieved using the gamma knife on targets within e.g. the brain.

Stereotactic radiation surgery is a minimally invasive treatment modality that allows delivery of a large single dose of radiation to a specific intracranial target while sparing surrounding tissue. Unlike conventional fractionated radiation therapy, stereotactic radiation surgery does not rely on, or exploit, the higher radiation sensitivity of neoplastic lesions relative to normal brain (therapeutic ratio). Its selective destruction depends primarily on sharply focused high-dose radiation and a steep dose gradient around the defined target. The biological effect is irreparable cellular damage and delayed vascular occlusion within the high-dose target volume. Because a therapeutic ratio is not required, traditionally radiation resistant lesions can be treated. Because destructive doses are used, however, any normal structure included in the target volume is subject to damage.

In radiation therapy system such as in a LINAC or Leksell Gamma Knife^{®}, the head of a patient is immobilized in a stereotactic instrument, which defines the location of the treatment volume in the head. Further, the patient is secured in a patient positioning unit, which moves the entire patient so as to position the treatment volume in coincidence with the focus point of the radiation unit of the radiation therapy system. Consequently, in radiation therapy systems, such as a LINAC system or a Leksell Gamma Knife^{®} system, it is of a high importance that the positioning unit is capable of position the treatment volume in coincidence with the focus point at a very high precision. This high precision must also be maintained over time.

Hence, in order to obtain as favorable clinical effect as possible during the therapy and to avoid damages to the surrounding tissue is it of an utmost importance that the radiation reaches and hits the target, i.e. the treatment volume, with a high precision and thereby spares the healthy tissue being adjacent to and/or surrounding the treatment volume. To achieve this, the patient must be immobilized during a therapy session and, moreover, the position of the head, or the part of the patient being under treatment, must be the same in a therapy session as in a reference position, i.e. the position during the session when the pictures to create the therapy plan were captured by means of, for example, Computerized Tomography Imaging (CT-imaging). For example, when the treatment area or volume is a portion of tissue within the head of a patient, a face mask adapted and shaped to be placed over the face (and shoulders) of the patient to thereby keep the patient in a substantially fixed position relative to the positioning system is used.

It is of particular importance to secure that the patient does not move during the delivery of the radiation therapy when conducting radiation surgery of tumors in proximity to sensitive tissue.

In the different fixation devices used today it is not possible to immobilize that patient to such an extent that motions of body parts of the patient is completely eliminated or prevented. For example, even though the patient is fixated using a face and shoulder mask there is a possibility of small motions of the head beneath the mask.

In light of this, there is a need within the art of radiation therapy for intra-fraction motion management (IFMM) systems and methods that enable detection and monitoring of very small motions of the patient and /or the treated body part with a high degree of accuracy and reliability during the therapy.

In the prior art, there exists a number of solutions for intra-fraction motion management (IFMM) based on, for example, X-ray imaging, optical imaging or invasive solutions. Such tracking systems are described e.g. in documents US 5622187, WO 2010/012983 and WO 98/17177. However, these prior art methods are associated with drawbacks. For example, imaging methods such as X-ray imaging or optical imaging require extensive image processing which may lead to complex and expensive solutions. X-ray imaging also exposes the patient for radiation, which may be injurious. Invasive solutions may be uncomfortable for the patient and may also be injurious for the patient. Furthermore, the prior art systems may have problems in withstanding the gamma radiation generated in, for example, a Perfexion^{®} system (a radiation therapy system provided by the applicant). Further, the prior art systems are often bulky which makes it difficult to use them together with, for example, the Perfexion^{®} system.

Another solution is to provide IR-markers on the mask and detect the movements by means of an IR-tracking system, for example, an IR camera. However, it is not possible to track small motions of the target beneath the mask, e.g. the head of the patient, since only movements of the mask itself will be tracked in this solution. For the same reason, surface tracking IFMM systems such as the C-rad catalyst are not suitable.

Thus, there is a need within the art of radiation therapy for improved systems and methods that enable intra-fraction motion detection with a high degree of accuracy and reliability, so as to avoid or at least significantly reduce the risk of undesired damages to surrounding sensitive tissue. These systems should not increase the complexity of the system and the treatment process itself.

### Summary of the invention

An object of the present invention is to provide improved systems for intra-fraction motion detection with a high degree of accuracy and reliability so as to avoid or at least significantly reduce the risk of undesired damages to surrounding sensitive tissue.

A further object of the present invention is to provide improved systems for intra-fraction motion detection that easily can be integrated into or be used together with a radiation therapy system such as the Perfexion^{®} system.

Yet another object of the present invention is to provide improved systems for intra-fraction motion detection that can be manufactured at a low cost.

Still another object of the present invention is to provide improved systems for intra-fraction motion management that are user-friendly and hence are easy to use for the medical personnel handling the radiation therapy system.

Another object of the present invention is to provide improved systems for intra-fraction motion management that are comfortable for the patient during use thereof.

A further object of the present invention is to provide improved systems for intra-fraction motion management that are compatible with imaging methods such as Computerized Tomography Imaging (CT-imaging) or Cone Beam Computerized Tomography Imaging (CBCT-imaging).

Another object of the present invention is to provide non-ionization systems for intra-fraction motion management (i.e. motion detection during the treatment sessions).

These and other objects are achieved by providing systems having the features defined in the independent claim. Preferred embodiments are defined in the dependent claims.

The systems according to the present invention are preferably used for monitoring intra-fraction motions of a patient in connection with treatment of cancer tumors of the patient in a radiation therapy system such as the Perfexion^{®} system. There is provided a system for monitoring intra-fraction motions of a patient in connection with treatment of treatment volumes such as cancer tumors of the patient in a radiation therapy system, which radiation therapy system comprises a radiation therapy unit having a radiation target and a patient positioning unit for positioning a treatment volume in a patient in relation to the target in the radiation therapy unit. The system comprises at least one patient marker arranged to be attached to the patient and at least one reference marker which establishes the origin of a local coordinate system. The at least one reference marker is preferably arranged to be positioned in a fixed position relative to a patient fixation arrangement for fixation of the patient during treatment. An optical tracking system is arranged in a position such that images of the patient marker and the reference markers can be captured. A processing module is configured to determine a relative shift in position from the initial starting position within the coordinate system established by the reference marker. The movement is determined based on images captured by the optical tracking system, wherein any relative shift indicates that the patient or a part of the patient has moved

The optical tracking system is preferably arranged in a position such that images of the patient marker, when the marker is provided on the nose of the patient, and at least one reference marker can be captured in an image. The optical tracking system is placed so that all markers are within the tracking volume in a position where all markers remain in view during the treatment session.

The present invention is based on the insight that a movement of a patient marker provided on the nose tip of the patient indicates movement of the head. The nose is a part of the face that is not intended to be moved and is therefore not connected to a lot of muscles, like most other parts of the face. Movements of the nose are normally very small and temporary, i.e. any part of the nose that is being moved normally comes back to its original position quickly. Furthermore, the motion tracking of present invention is based on changes on the position of the patient marker relative to a coordinate system of a reference indicator (preferably a reference tool including the reference marker(s)). The reference indicator is preferably firmly attached to a fixation arrangement for fixating the patient. The optical tracking system is arranged such that both the patient marker and the reference indicator are captured in an image. During the treatment, motions of the patient are tracked by the optical tracking system by monitoring changes in the position of the patient marker relative to the reference indicator. That is, by continuously obtaining images of the patient (i.e. the patient marker) and the reference markers (for example arranged on a reference tool) at predetermined intervals and analyzing these images, patient motions can be detected and tracked over time.

According to embodiments of the present invention, the patient marker and the reference marker(s) are reflective and the optical tracking system is an IR tracking system including an IR emitter and an IR detector, for example, an IR camera. Thus, the emitted IR light is reflected by the markers and can be captured by the IR detector. The markers can be identified in the images as light spots and these positions can then be used for determining position changes.

In embodiments of the present invention, each of the patient marker and the reference marker(s) comprises an IR emitter and the optical tracking system includes an IR detector, for example, an IR camera. Hence, the light emitted from the markers can be captured by the IR detector. The markers can be identified in the images as light spots and these positions can then be used for determining position changes.

According to embodiments of the present invention, the optical tracking system comprises a camera. In this embodiment, the camera will capture the markers in each image. The markers can be identified in the images, for example, using image analysis. According to embodiments of the present invention, a reference coordinate system origin at a reference tool including the reference markers is determined. Based on this reference point, the position of the patient marker within the coordinates determined by the reference tool is then calculated. Hence, the relative distance between subsequent patient marker positions is based on the position of the patient marker and the position of the reference coordinate system in the images. Thereby, a reference coordinate system is created, which enables tracking of the patient marker in three dimensions. However, for example, during the tracking only the magnitude of the vector may be used.

According to embodiments of the present invention, in connection with a treatment session, the patient is provided with a mask shaped to fit the face of the patient. At a treatment session, the mask is placed on the face. The mask has a hole such that the nose of the patient is free, i.e. not covered by the mask when placed on the face of the patient and is able to move without touching the mask. Thereby, the patient marker can easily be attached to the nose tip of the patient directly on the skin of the patient.

According to embodiments of the present invention, the reference tool is configured to be mounted at the patient fixation arrangement in a fixed position. The reference tool comprises one or more reference markers (in embodiments the reference tool includes one, three, four or five markers but, however, more than five is of course conceivable) and is positioned in a fixed position relative the patient and the patient positioning unit. The reference tool may be attached to the patient fixation arrangement via a stand. In embodiments, the reference tool is removable and can thus be mounted to the patient fixation arrangement during treatment periods when the reference tool is actually used and can be removed when not used. In other embodiments, the reference tool is firmly attached to the patient fixation arrangement.

According to an embodiment of the present invention, first and second reference tools are mounted at fixed positions relative to said patient fixation arrangement, wherein each reference tool includes at least one of said reference markers. Put differently, the reference tool comprises first and second reference tool members, each comprising at least one of said reference markers. Consequently, at least two reference markers are positioned in defined positions relative to said patient fixation arrangement. Thus, the processing module is configured to determine a position of the patient marker relative to the coordinate system established by the at least two reference markers based on images captured by the optical tracking system. One or more of the reference tools or reference tool members may comprise two or more reference markers. The two or more reference markers may be arranged at different positions in the z-direction, i.e. in the lengthwise direction of the patient, such that the reference markers are at different distances from the optical tracking system. This difference in distance may be achieved by means of the shape of the reference tool(s), e.g. the reference tool(s) being curved or having one or more protruding portions extending in the z-direction to which reference marker(s) are attached, and/or by countersinking reference marker(s), i.e. mounting the reference marker(s) in a recess or blind hole. The two or more reference markers may also be arranged at different positions in the y-direction. The reference tools or reference tool members may furthermore be spaced apart in the x-direction. Having reference markers arranged spaced apart in two or more directions is advantageous to achieve a more precise determination of the position of the patient marker. The first and second reference tools may be adapted to be mounted at said patient fixation arrangement. Alternatively, the first and second reference tools may be adapted to be mounted at said patient positioning unit. The reference tools may furthermore be adapted to be mounted on opposite sides of the head or neck of said patient.

According to another embodiment of the present invention, the head of the patient is fixed using a stereotactic head frame instead of using a mask. The head frame is adapted to be mounted at the patient fixation arrangement. In this embodiment, the reference marker(s) may be fixed directly to the head frame, i.e. without the use of one or more separate reference tools. The head frame may comprise two or more reference markers. The two or more reference markers may be arranged at different positions in the z-direction, i.e. in the lengthwise direction of the patient, such that the reference markers are at different distances from the optical tracking system. This difference in distance may be achieved by means of the shape of the head frame, e.g. the head frame having a curved portion or having one or more protruding portions extending in the z-direction to which reference marker(s) are attached, and/or by countersinking reference marker(s), i.e. mounting the reference marker(s) in a recess or blind hole. The two or more reference markers may also be arranged at different positions in the y-direction and/or in the x-direction. Having reference markers arranged spaced apart in two or more directions is advantageous to achieve a more precise determination of the position of the patient marker.

According to embodiments of the present invention, the position of the patient marker relative to the reference tool is determined based on images captured by the optical tracking system, wherein changes in the relative position indicates that the patient or a part of the patient has moved.

According to embodiments of the present invention, the relative distance (between initial and actual position of the patient marker) is presented on a presentation device and is continuously updated to allow an operator of the system to view an indication of patient motions.

According to embodiments of the present invention, an interrupting signal is provided instructing the radiation therapy system to interrupt the treatment if a position change exceeding a predetermined limit and/or lasting at least a predetermined period of time is detected. Thereby, the treatment can be immediately interrupted if the patient has moved such that the therapy volume, e.g. a cancer tumor, has been moved from the initial treatment position more than allowed, and hence potential damage to surrounding tissue can be avoided.

According to embodiments of the present invention, an alert signal is provided if a position change exceeding a predetermined limit and/or lasting at least a predetermined period of time is detected. Thereby, the medical personnel handling the radiation therapy system can be informed that the patient has moved such that the therapy volume, e.g. a cancer tumor, has been moved from the initial treatment position, which hence may cause damage to surrounding tissue. The alert signal thus notifies the medical personnel that the therapy may have to be interrupted and the patient repositioned before that therapy session is resumed. In embodiments of the present invention, the alert signal may be an audible signal or a visible signal and message and may be an electrical signal to the control unit to interrupt the therapy.

According to embodiments of the present invention, a distance or time interval change is determined to be a detected change if the change exceeds a predetermined limit and/or lasts at least a predetermined time interval. Thereby, small motions associated, for example, with respiration or slightly larger motions during a short time, for example if the patient wrinkles his nose, that do not influence the therapy or cause damage to surrounding tissue can be filtered out and only motions that are large enough and/or are persistent are detected.

Further objects and advantages of the present invention will be discussed below by means of exemplifying embodiments.

These and other features, aspects and advantages of the invention will be more fully understood when considered with respect to the following detailed description, appended claims and accompanying drawings.

### Brief description of the drawings

The drawings are not necessarily drawn to scale and illustrate generally, by way of example, but no way of limitation, various embodiments of the present invention. Thus, exemplifying embodiments of the invention are illustrated by way of example and not by way of limitation in the figures of the accompanying drawings in which like references indicate similar elements. It should be noted that references to "an" or "one" embodiment in this discussion are not necessarily to the same embodiment, and such references mean at least one.

Preferred embodiments of the invention will now be described in greater detail with reference to the accompanying drawings, in which
Fig. 1 illustrates the general principle of a radiation therapy system in which the present invention may be used;
Fig. 2 illustrates the positioning unit used in the system of Fig. 1;
Fig. 3 illustrates a part of the positioning unit including the engagement points for holding a fixation interface unit in more detail;
Fig. 4 illustrates a system according to embodiments of the present invention;
Fig. 5 is a flow chart illustrating steps of a method for monitoring intra-fraction motions of a patient;
Figs. 6a and 6b illustrate an optical tracking system mounted to the patient positioning unit according to an embodiment of the present invention; Fig. 7a and 7b illustrate embodiments of the system according to the present invention;
Fig. 8 is a flow chart illustrating steps of a method for monitoring intra-fraction motions of a patient;
Figs. 9a and 9b illustrate an embodiment of the present invention in which two reference tools are mounted to the patient fixation arrangement;
Figs. 10a and 10b illustrate an embodiment of the present invention in which a head frame including reference markers is mounted to the patient fixation arrangement; and
Fig. 11 illustrates an embodiment of the present invention in which two reference tools are mounted to the patient positioning unit.

### Description of preferred embodiments

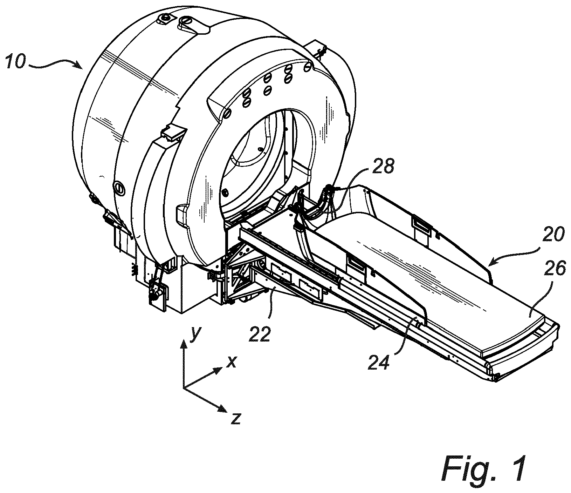

First, with reference to figs 1-3, a radiation therapy system for which the present invention is applicable comprises a radiation therapy unit or radiation unit 10 and a patient positioning unit 20 will be described. In the radiation unit 10, there are provided radioactive sources, radioactive source holders, a collimator body, and external shielding elements. The collimator body comprises a large number of collimator channels directed towards a common focus point, in a manner as is commonly known in the art.

The collimator body also acts as a radiation shield preventing radiation from reaching the patient other than through the collimator channels. Examples of collimator arrangements in radiation therapy systems applicable to the present invention can be found in US Patent No. 6,931,096. However, the present invention is also applicable to radiation therapy systems using other arrangements for collimating radiation into a focus point, such as disclosed in US Patent No. 4,780,898.

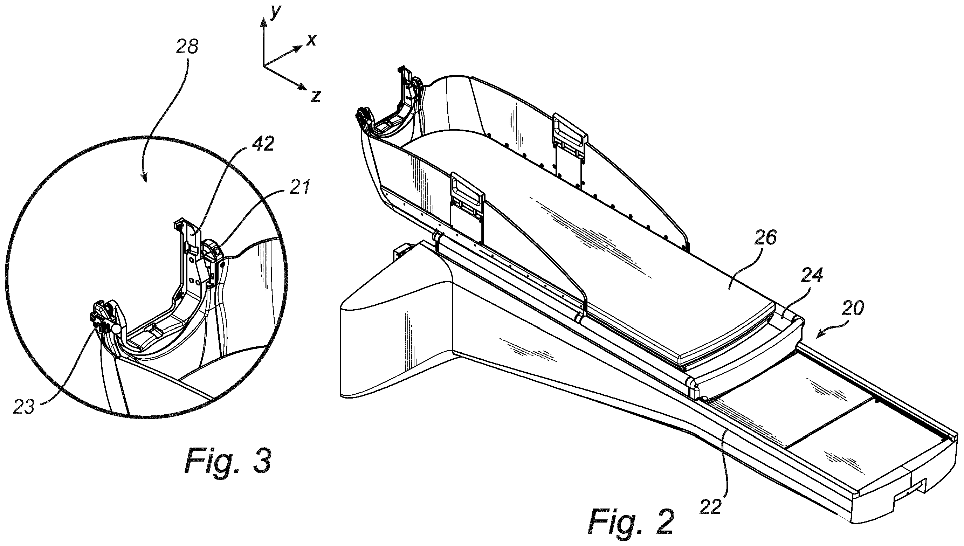

The patient positioning unit 20 comprises a rigid framework 22, a slidable or movable carriage 24, and motors (not shown) for moving the carriage 24 in relation to the framework 22. The carriage 24 is further provided with a patient bed 26 for carrying and moving the entire patient. At one end of the carriage 24, there is provided a fixation arrangement 28 for receiving and fixing a patient fixation unit or interface unit, either directly or via an adaptor unit 42, see Fig. 3.

The coordinates of the fixation unit are defined by a fixation unit coordinate system, which through the fixed relationship with the treatment volume also is used for defining the outlines of the treatment volume. In operation, the fixation unit, and hence the fixation unit coordinate system, is moved in relation to the fixed radiation focus point such that the focus point is accurately positioned in the intended coordinate of the fixation unit coordinate system.

The fixation arrangement 28 comprises two engagement points 21, 23, which are arranged for preventing the patient fixation unit from translational and/or rotational movement in relation to the movable carriage 24.

As can be understood from Figs. 1 and 2, the described embodiment concerns a radiation therapy system for providing gamma radiation therapy to a target volume in the head of human patient. Such therapy is often referred to as stereotactic radiation surgery. During therapy, the patient head is fixed in a fixation unit in the form of a stereotactic head frame, which comprises engagement points adapted for engagement with the engagement points 21, 23 of the radiation therapy system. Thus, during the stereotactic radiation surgery, the head of the patient is fixed in the stereotactic frame, which in turn is fixedly attached to the patient positioning unit via the engagement points 21, 23. During movement of the treatment volume in the head of the patient in relation to the radiation focus point, along the three orthogonal axes x, y, and z shown in Fig. 1, the entire patient is moved along the axes. Thus, there is no relative movement between the head frame and the carriage 24 of the patient positioning unit 20.

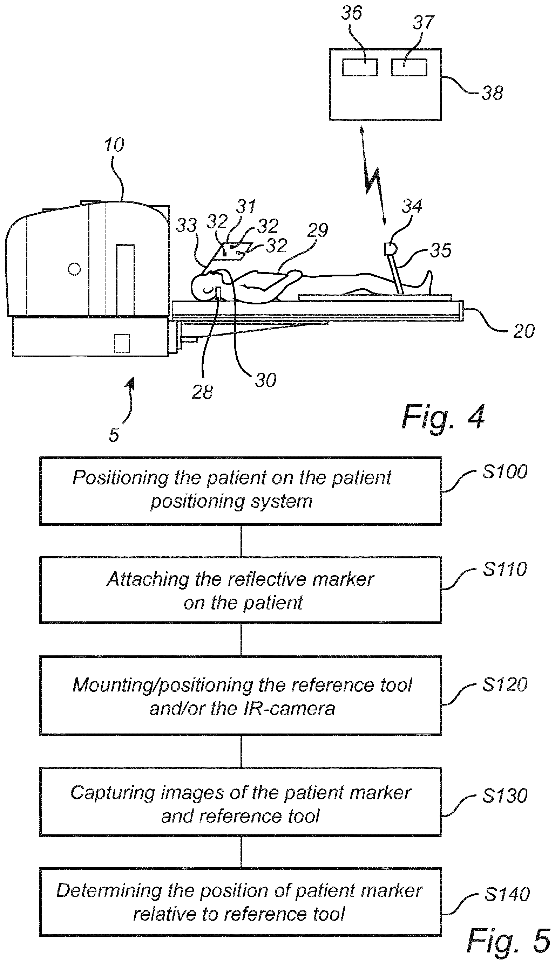

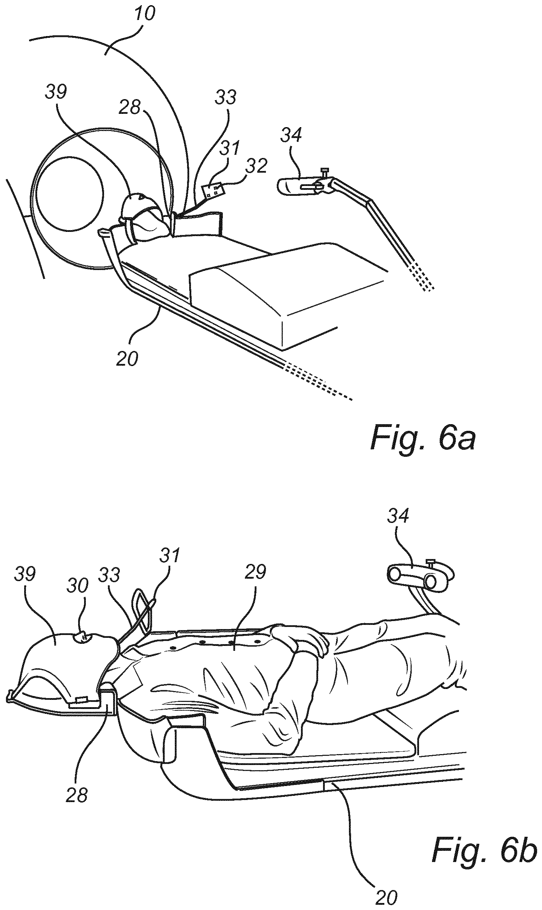

Turning now to Fig. 4, an embodiment of a system for intra-fraction motion detection and monitoring according to the present invention will be discussed. The intra-fraction motion detection system 5 comprises at least one patient marker 30 placed and positioned relative to the positioning unit 20 on the nose of the patient 29 when placed in the positioning system 20. The patient is provided with mask 39 (see Fig. 6b) having a hole at the nose allowing that the marker 30 is placed on the nose of the patient 29. The patient marker 30 is preferably reflective patch of single use type with biocompatible adhesive.

Furthermore, a reference tool 31 comprising reference markers 32 is positioned in a fixed position relative to the patient fixation arrangement 28 and the patient positioning unit 20. In Fig. 6a and 6b, a reference tool 31 comprising three markers 32 is shown mounted to the patient fixation arrangement 28. The reference tool 31 may be attached to the patient fixation arrangement 28 via a stand 33. In embodiments, the reference tool 31 is removable and can thus be mounted to the patient fixation arrangement 28 during treatment periods when the reference tool 31 is actually used and can be removed when not used. In other embodiments, the reference tool 31 is firmly attached to the patient fixation arrangement 28.

An optical tracking system 34 is arranged in a position such that images of the patient marker 30, when the marker is provided on the nose of the patient 29, and the reference markers 32 of the reference tool 31 can be captured.

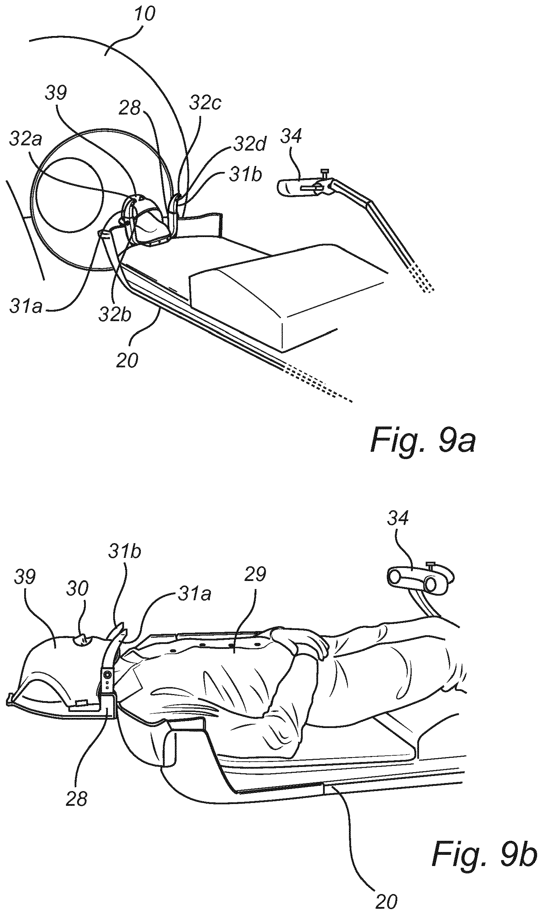

In Fig. 9a and 9b, an alternative embodiment is shown. A first reference tool 31a and a second reference tool 31b, each comprising two reference markers 32a-b and 32c-d, are mounted to the patient fixation arrangement 28, and thus in fixed positions relative to the patient positioning unit 20. The reference tools 31a-b are curved in the z-direction, i.e. in the lengthwise direction of the patient. Put differently, the reference tools are curved towards the optical tracking system 34 such that upper portions of the reference tools are closer to the optical tracking system than lower portions thereof. The reference tools 31a-b are mounted to the patient fixation arrangement 28 on opposite sides of the head and/or neck of the patient. Each reference tool comprises a first reference marker 32a, 32c attached to an upper portion or tip thereof, and a second reference marker 32b, 32d at a lower portion of said reference tool, i.e. closer to the patient positioning unit 20 when the reference tool is mounted to the patient fixation arrangement 28. Due to the curved shape of the reference tools, the first reference marker of each reference tool will be at a closer distance from the optical tracking system 34 than the second reference marker. In the embodiment shown in Fig. 9a and 9b, the second reference markers 32b, 32d are furthermore countersunk in the respective reference tool, i.e. mounted in a recess or blind hole, such that the difference in distance from the first and second reference markers to the optical tracking system is further increased. The reference tools may be removable and can thus be mounted to the patient fixation arrangement 28 during treatment periods when the reference tools are actually used and can be removed when not used. Alternatively, the reference tools may be firmly attached to the patient fixation arrangement 28. An optical tracking system 34 is arranged in a position such that images of the patient marker 30, when the marker is provided on the nose of the patient 29, and the reference markers 32a-d of the reference tool 31 can be captured.

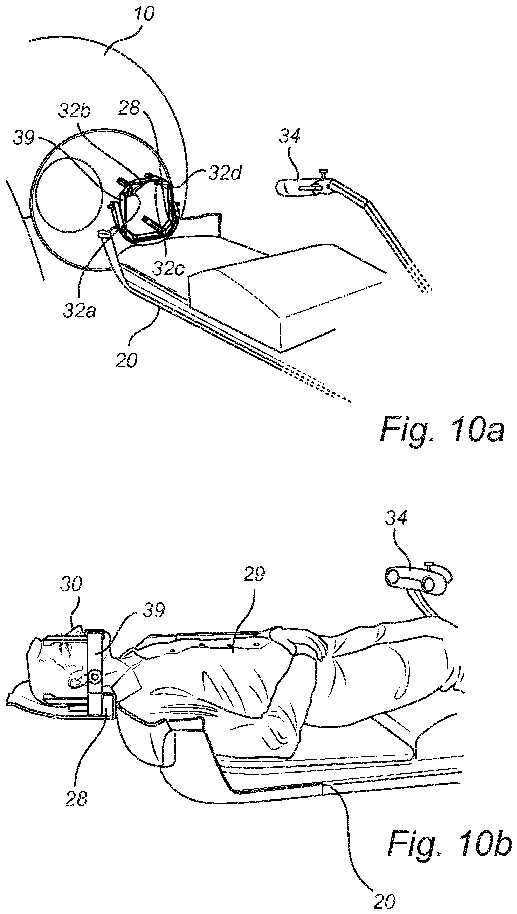

In Fig. 10a and 10b, another embodiment is shown. In this embodiment, the patient is fixed to the patient fixation arrangement 28 using a stereotactic head frame 39. Four reference markers 32a-d are attached directly to the head frame in a symmetric manner. The two outer reference markers 32a, 32d are spaced apart in all three directions (X, Y and Z) from the inner two reference markers 32b, 32c. The two outer reference markers 32a, 32d are arranged closer to the optical tracking system than the two inner reference markers 32b, 32c. At least one patient marker 30 is placed and positioned relative to the positioning unit 20 on the nose of the patient 29 when placed in the positioning system 20. As can be seen in Figs. 10a-b, the head frame is configured to not obstruct the light path between the patient marker 30 and the optical tracking system 34. The patient marker 30 is preferably a reflective patch of single use type with biocompatible adhesive. An optical tracking system 34 is arranged in a position such that images of the patient marker 30, when the marker is provided on the nose of the patient 29, and the reference markers 32 of the reference tool 31 can be captured.

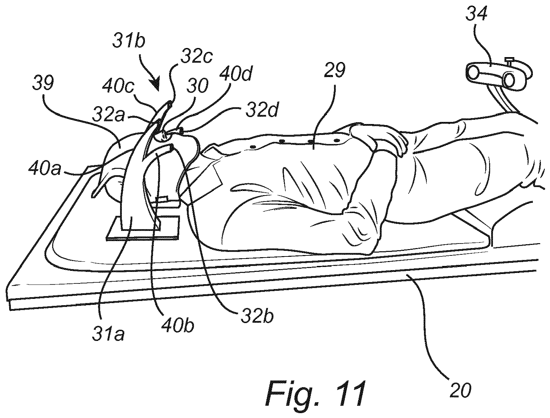

In Fig. 11, yet another embodiment is shown. A first reference tool 31a and a second reference tool 31b, each comprising two reference markers 32a-b and 32c-d, are mounted to the patient positioning unit 20. The reference tools 31a-b are mounted to the patient fixation arrangement 28 on opposite sides of the head/neck of the patient. Each reference tool comprises an upper protrusion 40a, 40c and a lower protrusion 40b, 40d. The protrusions are curved in the z-direction, i.e. in the lengthwise direction of the patient, thus towards the optical tracking system 34. Reference markers 32a-d are attached to the tip of each protrusion. The protrusions are configured such that the lower reference markers 32b, 32d are closer to the optical tracking system 34 than the upper reference markers 32a, 32c. An optical tracking system 34 is arranged in a position such that images of the patient marker 30, when the marker is provided on the nose of the patient 29, and the reference markers 32a-d of the reference tool 31 can be captured.

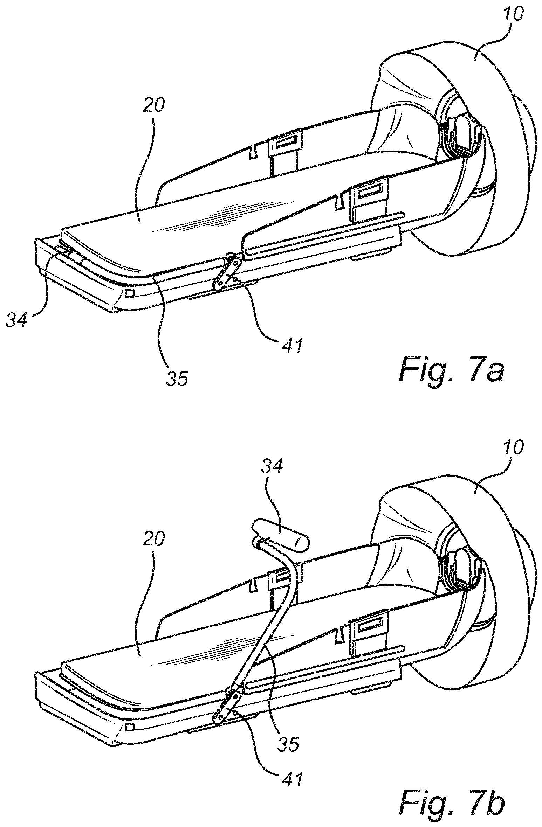

The optical tracking system 34 is arranged in a position relative the patient positioning unit 20. In embodiments of the present invention, the optical tracking system can be mounted to the patient positioning unit 20, for example, via a camera stand 35 attached to the patient positioning unit 20 using connection means 41 (see Fig. 7b) such as a hinge or the like, which enable a user to position and lock the optical tracking system in a desired position (see also Fig. 6a, 6b, 7a and 7b). According to embodiments of the present invention, the patient marker 30 is reflective and the optical tracking system is an IR tracking system including an IR emitter and an IR detector, for example, an IR camera. In other embodiments of the present invention, each of the patient marker and at least one reference marker comprises an IR emitter and the optical tracking system includes an IR detector, for example, an IR camera. According to further embodiments of the present invention, the optical tracking system comprises a camera. Suitable tracking systems are manufactured, for example, by NDI Recognition Systems Inc. In Figs. 7a and 7b, an IR tracking system 34 according to embodiments of the present invention is shown in a folded down position (Fig. 7a) and a folded up position (Fig. 7b).

Camera data such as, for example, data representing captured images are processed in a processing module 37 configured to determine the position of the patient marker relative to the coordinate system of the reference tool 31 and based on images captured by the optical tracking system and changes in that position indicates that the patient or a part of that patient has moved. The reference tool, or at least one reference marker, is used to determine the origin of a coordinate system. Both the patient marker and the reference tool (i.e. at least one reference marker) are seen by the camera and captured in images. Based on these images, the patient marker's position is calculated within this coordinate system by performing the relevant transformations on the information provided by the camera.

The processing module 37 may be arranged in an external unit 38, such as a personal computer or laptop, and image data can be transferred from the camera 34 to a communication module 36 of the external unit 38 wirelessly, e.g. using Bluetooth, or via cable. The processing module 37 may be implemented as a software module arranged to be executed on a computer unit such as a laptop or personal computer.

With reference now to Fig. 5, the general principles of a method for monitoring intra-fraction motions of a patient will be discussed. The method can be used for intra-fraction motion detection and monitoring, for example, in therapy sessions during fractionated radiation therapy in connection with treatment of cancer. When the method has been initiated, the method is preferably continued during the whole treatment session so as to monitor patient movements throughout the session.

First, in step S100, the patient 29 is placed on the patient positioning unit 20 and is positioned such that a treatment volume, e.g. the cancer tumor, is positioned in a treatment position in relation to the target volume in the radiation therapy unit 10. In this example, the patient is provided with a mask shaped to fit the face of the patient. The mask is provided with a hole such that the nose of the patient is free, i.e. not covered by the mask and such that the mask does not affect the movement of the nose so that the nose moves with the target, when the mask is placed on the face of the patient. Thereby, it is easy to attach the patient marker directly on the nose tip of the patient and, at step S110, the patient marker 30 is attached on the nose of the patient. Preferably, the patient marker 30 is attached on the nose tip of the patient. Other methods for fixating the patient relative to the patient positioning unit 20 include invasive fixation or fixation using a bite-block.

At step S120, the reference tool 31 is mounted firmly to the patient fixation arrangement 28 such that the reference tool 31 is fixed relative to the patient positioning unit 20 in a desired position. If the reference tool 31 is not removable or if the reference tool 31 already is mounted to the patient fixation arrangement 28, this is not necessary. Further, the optical tracking system 34 is positioned (e.g. placed in a folded up position) in a position relative the patient and the reference tool such that both the patient marker 30 and the markers 32 of the reference tool 31 can be captured in an image.

At step S130, during the treatment, images of the patient marker 30 and reference markers of the reference tool 31 are repeatedly captured at predetermined time intervals.

At step S140, a position of the patient marker relative to its initial position is continuously determined based on the images captured by the optical tracking system 34. Changes in that distance indicate that the patient or a part of the patient has moved relative the patient positioning unit 20. Preferably, the position of the patient marker relative to the reference tool is determined based on images captured by the optical tracking system 34.

The patient marker movement can be presented on a presentation device during the treatment for an operator of the radiation therapy unit 10.

The steps S130 and S140 are continuously repeated during the therapy session until the session has been terminated.

During the treatment, it may be checked whether an observed motion exceeds a predetermined limit and/or lasts at least a predetermined period of time, i.e. whether the patient marker movement exceeds a predetermined limit and/or lasts at least a predetermined period of time. If a motion is observed that exceeds the predetermined limit and/or lasts the predetermined period of time, the treatment session may be interrupted and/or an alert signal may be issued.

The external unit 38 may send an interruption signal to the radiation therapy system 10 instructing it to immediately interrupt the treatment. Thereby, it is secured that potential damage to surrounding tissue is minimized.

If an alert signal is issued, the alert signal may be an audible and/or visible signal. Thereby, the medical personnel performing the therapy are informed and alerted of the fact that the patient has moved from his initial therapy position, which may lead to impaired therapy, and may take proper actions. The treatment could also be automatically interrupted.

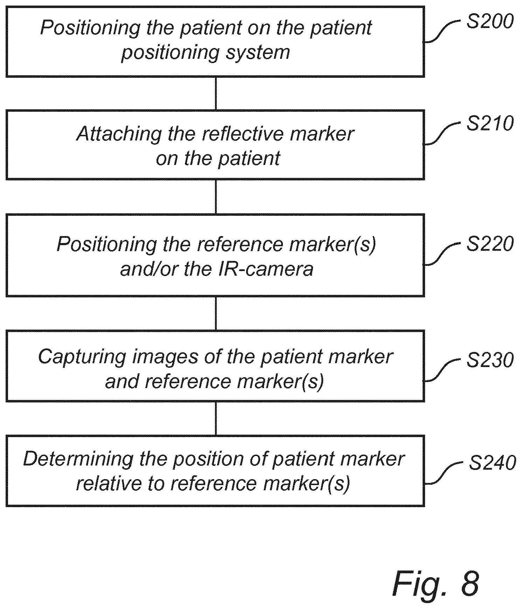

With reference now to Fig. 8, the general principles of a method for monitoring intra-fraction motions of a patient will be discussed. The method can be used for intra-fraction motion detection and monitoring, for example, in therapy sessions during fractionated radiation therapy in connection with treatment of cancer. When the method has been initiated, the method is preferably continued during the whole treatment session so as to monitor patient movements throughout the session.

First, in step S200, the patient 29 is placed on the patient positioning unit 20 and is positioned such that a treatment volume, e.g. the cancer tumor, is positioned in a treatment position in relation to the target volume in the radiation therapy unit 10. In this example, the patient is provided with a mask shaped to fit the face of the patient. The mask is provided with a hole such that the nose of the patient is free, i.e. not covered by the mask and such that the mask does not affect the movement of the nose so that the nose moves with the target, when the mask is placed on the face of the patient. Thereby, it is easy to attach the patient marker directly on the nose tip of the patient and, at step S210, the patient marker 30 is attached on the nose of the patient. Preferably, the patient marker 30 is attached on the nose tip of the patient. Other methods for fixating the patient relative to the patient positioning unit 20 include invasive fixation or fixation using a bite-block. One method for fixating the patient relative to the patient positioning unit 20 is to use a stereotactic head frame, as shown in figures 10a-b and explained in further detail below.

At step S220, the reference marker(s) are positioned at fixed positions relative to the patient fixation arrangement 28 and/or the patient positioning unit 20. This may be achieved by mounting one or more reference tools, each including one or more reference marker, at the patient fixation arrangement 28 or directly to the patient positioning unit 20. If the reference tool 31 is not removable or if the reference tool(s) 31 is already mounted to the patient fixation arrangement 28, this is not necessary. Other methods for positioning the reference marker(s) include using a stereotactic head frame on which the reference marker(s) are arranged. If such a head frame is already mounted, no further action is needed to position the reference marker(s).

Further, the optical tracking system 34 is positioned (e.g. placed in a folded up position) in a position relative the patient marker and the reference marker(s) such that both the patient marker 30 and the markers 32 of the reference tool 31 can be captured in an image.

At step S230, during the treatment, images of the patient marker 30 and reference markers are repeatedly captured at predetermined time intervals.

At step S240, a position of the patient marker relative to its initial position is continuously determined based on the images captured by the optical tracking system 34. Changes in that distance indicate that the patient or a part of the patient has moved relative the patient positioning unit 20. Preferably, the position of the patient marker relative to the reference marker(s) is determined based on images captured by the optical tracking system 34.

The patient marker movement can be presented on a presentation device during the treatment for an operator of the radiation therapy unit 10.

The steps S230 and S240 are continuously repeated during the therapy session until the session has been terminated.

## Claims

1. A system (5) for monitoring intra-fraction motions of a patient in connection with treatment of treatment volumes such as cancer tumors of said patient in a radiation therapy system, which radiation therapy system comprises an external beam radiation therapy unit having a radiation focus point and a patient positioning unit for positioning a treatment volume in a patient in relation to said focus point in the radiation therapy unit, said system comprising:
a patient fixation arrangement (28) for fixation of said patient during treatment;
at least one patient marker (30) arranged to be attached to the nose of the patient such that movement of the patient marker (30) indicates movement of the head;
at least one reference marker (32) arranged to be positioned in a defined position relative to the patient fixation arrangement (28);
an optical tracking system (34) arranged in a position such that images of the at least one patient marker, when said at least one patient marker (30) is provided on the nose of the patient, and of the at least one reference marker (32) can be captured, said position of said optical tracking system (34) being fixed relative to the at least one reference marker (32); and
a processing module (37) configured to determine a position of the at least one patient marker (30) relative to the coordinate system established by the at least one reference marker (32) based on images captured by the optical tracking system (34), wherein changes in said position provides information if the patient or a part of said patient has moved relative to said patient fixation arrangement (28), wherein the processing module (37) is configured to provide an interrupting signal instructing the radiation therapy system to interrupt the treatment if a motion change exceeding a predetermined limit and/or lasting at least a predetermined period of time is detected.

2. The system according to claim 1, wherein the patient marker is reflective and the optical tracking system is an IR tracking system including an IR emitter and an IR detector.

3. The system according to claim 1, wherein each of the patient marker and the at least one reference marker comprises an IR emitter and the optical tracking system includes an IR detector.

4. The system according to claim 1, wherein the optical tracking system comprises a camera.

5. The system according to claim 1, wherein said processing module is configured to:
determine an average position of the reference point using said reference markers; and
determine a position of the patient marker relative a coordinate system determined by the reference point.

6. The system according to claim 1, wherein a reference tool including said at least one reference marker is arranged to be mounted at said patient fixation arrangement in a fixed position or a position defined relative said patient fixation arrangement.

7. The system according to claim 1, further comprising a presentation device arranged to present an indication of patient motions.

8. The system according to any one of claims 1 - 7, wherein said system comprises at least two reference markers arranged to be positioned in defined positions relative to said patient fixation arrangement, and wherein said processing module is configured to determine a position of the patient marker relative to the coordinate system established by the at least two reference markers based on images captured by the optical tracking system.

9. The system according to claim 8, further comprising first and second reference tools adapted to be mounted at fixed positions relative to said patient fixation arrangement, wherein each reference tool includes at least one of said reference markers.

10. The system according to claim 9, wherein said first and second reference tools are adapted to be mounted at said patient fixation arrangement.

11. The system according to claim 9, wherein said first and second reference tools are adapted to be mounted at said patient positioning unit.

12. The system according to any one of claims 9 - 11, wherein said reference tools are adapted to be mounted on opposite sides of the head or neck of said patient.

13. The system according to any one of claims 9 - 12, wherein at least one of said reference tools comprises at least two reference markers arranged to be at different distances from said optical tracking system when said reference tool(s) is mounted.

14. The system according to claim 8, further comprising a stereotactic head frame adapted to be mounted at said patient fixation arrangement, said head frame being adapted to fixate the head of said patient, wherein said head frame includes said at least two reference markers.

15. The system according to claim 14, wherein said at least two reference markers are arranged on said head frame to be at different distances from said optical tracking system when said head frame is mounted.

## Patentansprüche

1. System (5) zum Überwachen von bruchinternen Bewegungen eines Patienten in Verbindung mit einer Behandlung von Behandlungsvolumen, wie z. B. Krebstumoren des Patienten, in einem Bestrahlungstherapiesystem, wobei das Strahlentherapiesystem eine Externstrahlen-Bestrahlungstherapieeinheit mit einem Bestrahlungsbrennpunkt und eine Patientenpositionierungseinheit zum Positionieren eines Behandlungsvolumens in einem Patienten in Bezug auf den Brennpunkt der Bestrahlungstherapieeinheit umfasst, wobei das System umfasst:
eine Patientenfixieranordnung (28) für die Fixierung des Patienten während der Behandlung;
mindestens einen Patientenmarker (30), der dazu vorgesehen ist, so an der Nase des Patienten angebracht zu sein, dass eine Bewegung des Patientenmarkers (30) eine Bewegung des Kopfs anzeigt;
mindestens einen Referenzmarker (32), der dazu vorgesehen ist, in einer definierten Position relativ zu der Patientenfixieranordnung (28) positioniert zu sein;
ein optisches Verfolgungssystem (34), das so in einer Position angeordnet ist, dass Bilder des mindestens einen Patientenmarkers, wenn sich der mindestens eine Patientenmarker (30) auf der Nase des Patienten befindet, und des mindestens einen Referenzmarkers (32) erfasst werden können,
wobei die Position des optischen Verfolgungssystems (34) relativ zu dem mindestens einen Referenzmarker (32) festgelegt ist; und
ein Verarbeitungsmodul (37), das so ausgeführt ist, dass es eine Position des mindestens einen Patientenmarkers (30) relativ zu dem Koordinatensystem, das von dem mindestens einen Referenzmarker (32) gebildet wird, auf der Basis von Bildern bestimmt, die von dem optischen Verfolgungssystem (34) erfasst werden, wobei Veränderungen der Position Informationen darüber liefern, ob sich der Patient oder ein Teil des Patienten relativ zu der Patientenfixieranordnung (28) bewegt hat, wobei das Verarbeitungsmodul (37) so ausgeführt ist, dass es ein Unterbrechungssignal liefert, das das Bestrahlungstherapiesystem anweist, die Behandlung zu unterbrechen, wenn eine Bewegungsveränderung, die einen vorbestimmten Grenzwert überschreitet und/oder mindestens eine vorbestimmte Zeitperiode andauert, detektiert wird.

2. System nach Anspruch 1, wobei der Patientenmarker reflektierend ist und das optische Verfolgungssystem ein IR-Verfolgungssystem ist, das einen IR-Emitter und einen IR-Detektor aufweist.

3. System nach Anspruch 1, wobei der Patientenmarker und der mindestens eine Referenzmarker einen IR-Emitter umfassen und das optische Verfolgungssystem einen IR-Detektor aufweist.

4. System nach Anspruch 1, wobei das optische Verfolgungssystem eine Kamera umfasst.

5. System nach Anspruch 1, wobei das Verarbeitungsmodul so ausgeführt ist, dass es:
eine mittlere Position des Referenzpunkts unter Verwendung der Referenzmarker bestimmt; und
eine Position des Patientenmarkers relativ zu einem Koordinatensystem, das von dem Referenzpunkt bestimmt wird, bestimmt.

6. System nach Anspruch 1, wobei ein Referenztool, das mindestens einen Referenzmarker aufweist, dazu vorgesehen ist, in einer festgelegten Position oder einer Position, die relativ zu der Patientenfixieranordnung definiert ist, an der Patientenfixieranordnung angebracht zu sein.

7. System nach Anspruch 1, das ferner eine Darstellungsvorrichtung umfasst, die dazu vorgesehen ist, eine Anzeige von Patientenbewegungen darzustellen.

8. System nach einem der Ansprüche 1-7, wobei das System mindestens zwei Referenzmarker umfasst, die dazu vorgesehen sind, in definierten Positionen relativ zu der Patientenfixieranordnung positioniert zu sein, und wobei das Verarbeitungsmodul so ausgeführt ist, dass es eine Position des Patientenmarkers relativ zu dem Koordinatensystem, das von den mindestens zwei Referenzmarkern gebildet wird, auf der Basis der Bilder, die von dem optischen Verfolgungssystem erfasst werden, bestimmt.

9. System nach Anspruch 8, das ferner ein erstes und ein zweites Referenztool umfasst, die so ausgebildet sind, dass sie an festgelegten Positionen relativ zu der Patientenfixieranordnung angebracht sind, wobei jedes Referenztool mindestens einen der Referenzmarker aufweist.

10. System nach Anspruch 9, wobei das erste und das zweite Referenztool so ausgebildet sind, dass sie an der Patientenfixieranordnung angebracht sind.

11. System nach Anspruch 9, wobei das erste und das zweite Referenztool so ausgebildet sind, dass sie an der Patientenpositionierungseinheit angebracht sind.

12. System nach einem der Ansprüche 9-11, wobei die Referenztools so ausgebildet sind, dass sie auf gegenüberliegenden Seiten des Kopfs oder Halses des Patienten angebracht sind.

13. System nach einem der Ansprüche 9-12, wobei mindestens eines der Referenztools mindestens zwei Referenzmarker umfasst, die dazu vorgesehen sind, sich in unterschiedlichen Abständen zu dem optischen Verfolgungssystem zu befinden, wenn das (die) Referenztool(s) angebracht ist (sind).

14. System nach Anspruch 8, das ferner einen stereotaktischen Kopfrahmen umfasst, der so ausgebildet ist, dass er an der Patientenfixieranordnung angebracht ist, wobei der Kopfrahmen so ausgebildet ist, dass er den Kopf des Patienten fixiert, wobei der Kopfrahmen die mindestens zwei Referenzmarker aufweist.

15. System nach Anspruch 14, wobei die mindestens zwei Referenzmarker so an dem Kopfrahmen angeordnet sind, dass sie sich in unterschiedlichen Abständen zu dem optischen Verfolgungssystem befinden, wenn der Kopfrahmen angebracht ist.

## Revendications

1. Système (5) pour surveiller des mouvements intra-fraction d'un patient en liaison avec le traitement de volumes de traitement, tels que des tumeurs cancéreuses dudit patient dans un système radiothérapie, lequel système de radiothérapie comprend une unité de radiothérapie par faisceau externe ayant un point de focalisation du rayonnement et une unité de positionnement de patient pour positionner un volume de traitement d'un patient en liaison avec ledit point de focalisation dans l'unité de radiothérapie, ledit système comprenant :
un agencement de fixation de patient (28) destiné à la fixation dudit patient au cours du traitement ;
au moins un marqueur de patient (30) agencé pour être fixé au nez du patient de manière à ce que le mouvement du marqueur de patient (30) indique le mouvement de la tête ;
au moins un marqueur de référence (32) agencé pour être positionné dans une position définie par rapport à l'agencement de fixation de patient (28) ;
un système de suivi optique (34) agencé dans une position telle que des images de l'au moins un marqueur de patient, lorsque ledit au moins un marqueur de patient (30) est pourvu sur le nez du patient, et de l'au moins un marqueur de référence (32) peuvent être capturées,
ladite position dudit système de suivi optique (34) étant fixée par rapport à l'au moins un marqueur de référence (32) ; et
un module de traitement (37) configuré pour déterminer une position de l'au moins un marqueur de patient (30) par rapport au système de coordonnées établi par l'au moins un marqueur de référence (32) sur la base d'images capturées par le système de suivi optique (34), où des changements de ladite position fournissent des informations si le patient, ou une partie dudit patient, a bougé par rapport audit agencement de fixation de patient (28),
où le module de traitement (37) est configuré pour fournir un signal d'interruption donnant pour instruction au système de radiothérapie d'interrompre le traitement si un changement de mouvement excédant une limite prédéterminée et/ou s'étendant sur au moins une durée prédéterminée est détecté.

2. Système selon la revendication 1, dans lequel le marqueur de patient est réfléchissant et le système de suivi optique est un système de suivi infrarouge comprenant un émetteur infrarouge et un détecteur infrarouge.

3. Système selon la revendication 1, dans lequel chacun du marqueur de patient et de l'au moins un marqueur de référence comprend un émetteur infrarouge, et le système de suivi optique comprend un détecteur infrarouge.

4. Système selon la revendication 1, dans lequel le système de suivi optique comprend une caméra.

5. Système selon la revendication 1, dans lequel ledit module de traitement est configuré pour :
déterminer une position moyenne du point de référence au moyen desdits marqueurs de référence ; et
déterminer une position du marqueur de patient par rapport à un système de coordonnées déterminé par le point de référence.

6. Système selon la revendication 1, dans lequel un outil de référence comprenant ledit au moins un marqueur de référence est agencé pour être monté sur ledit agencement de fixation de patient dans une position fixe ou une position définie relativement audit agencement de fixation de patient.

7. Système selon la revendication 1, comprenant en outre un dispositif de présentation agencé pour présenter une indication des mouvements du patient.

8. Système selon l'une quelconque des revendications 1 à 7, dans lequel ledit système comprend au moins deux marqueurs de référence agencés pour être positionnés dans des positions définies par rapport audit agencement de fixation de patient, et où ledit module de traitement est configuré pour déterminer une position du marqueur de patient par rapport au système de coordonnées établi par les au moins deux marqueurs de référence sur la base des images capturées par le système de suivi optique.

9. Système selon la revendication 8, comprenant en outre des premier et second outils de référence adaptés pour être montés à des positions fixes par rapport audit agencement de fixation de patient, où chaque outil de référence comprend au moins un desdits marqueurs de référence.

10. Système selon la revendication 9, dans lequel lesdits premier et second outils de référence sont adaptés pour être montés au niveau agencement de fixation de patient.

11. Système selon la revendication 9, dans lequel lesdits premier et second outils de référence sont adaptés pour être montés au niveau de ladite unité de positionnement du patient.

12. Système selon l'une quelconque des revendications 9 à 11, dans lequel lesdits outils de référence sont adaptés pour être montés sur des côtés opposés de la tête ou du cou dudit patient.

13. Système selon l'une quelconque des revendications 9 à 12, dans lequel au moins un desdits outils de référence comprend au moins deux marqueurs de référence disposés de manière à être à des distances différentes dudit système de suivi optique lorsque ledit/lesdits outil(s) de référence sont montés.

14. Système selon la revendication 8, comprenant en outre un châssis de tête stéréotaxique adapté pour être monté au niveau dudit agencement de fixation de patient, ledit châssis de tête étant adapté pour fixer la tête dudit patient, où ledit châssis de tête comprend lesdits au moins deux marqueurs de référence.

15. Système selon la revendication 14, dans lequel lesdits au moins deux marqueurs de référence sont disposés sur ledit châssis de tête pour être à des distances différentes dudit système de suivi optique lorsque ledit châssis de tête est monté.
